# EUROPÄISCHE PATENTSCHRIFT

(11) **EP 0 830 098 B1**
(45) Veröffentlichungstag und Bekanntmachung des Hinweises auf die Patenterteilung: **22.03.2000**
(21) Anmeldenummer: 96916101.7
(22) Anmeldetag: 17.05.1996
(51) Int. Cl.: A61B 19/00

(54) **VORRICHTUNG UND VERFAHREN ZUR FIXIERUNG DES MENSCHLICHEN KOPFES**
METHOD AND DEVICE FOR FIXING THE HUMAN HEAD
PROCEDE ET DISPOSITIF POUR FIXER LA TETE HUMAINE

(30) Priorität: 18.05.1995 DE 29508277 U; 25.07.1995 DE 29511995 U
(43) Veröffentlichungstag der Anmeldung: 25.03.1998
(73) Patentinhaber: Vogele, Michael, 86830 Schwabmünchen (DE)
(72) Erfinder: Vogele, Michael, 86830 Schwabmünchen (DE)
(74) Vertreter: Fiener, Josef
(86) Internationale Anmeldenummer: EP9602109
(87) Internationale Veröffentlichungsnummer: WO9636292

(56) Entgegenhaltungen:
- WO-A-90/13257
- GB-A- 2 164 856
- GB-A- 2 213 066
- US-A- 4 602 622
- US-A- 5 230 623
- US-A- 5 464 411

## Beschreibung

Die Erfindung betrifft eine Vorrichtung und ein Verfahren zur Fixierung des menschlichen Kopfes.

In vielen Bereichen der Humanmedizin oder der medizinischen Forschung ist eine sichere Fixierung des Kopfes eines Patienten oder einer Untersuchungsperson erforderlich. Von größter Bedeutung ist dies insbesondere auf dem Gebiet der Kopfchirurgie und der Hals-/Nasen-/Ohrenchirurgie. Durch die Einbeziehung der bildgebenden Computertechnologie in Diagnose und Therapie sind die Anforderungen an Genauigkeit und Reproduzierbarkeit sowohl bei der Festlegung eines räumlich definierten Punktes im oder am menschlichen Kopf, als auch bei der Fixierung des Kopfes selbst, gestiegen.

Im folgenden werden die bekanntesten Methoden zur Fixierung des menschlichen Kopfes kurz beschrieben:
a) Fixation des Kopfes mit Klebeband:
   Der Kopf des Patienten liegt in Rückenlage auf einem Schaumstoffring. Quer über die Stirn und Ober-/Unterkiefer gespannte Klebebänder fixieren den Kopf auf eine Unterlage. Nachteilig sind dabei folgende Punkte:
   - Durch starken Zug der Klebebänder kann es zu Druckstellen, Verschiebungen und/oder zu Hautschwellungen kommen;
   - nach Abnahme der Halteelemente (Klebebänder) ist eine erneute Repositionierung in genau gleicher Lage kaum mehr möglich, was besonders bei stereotaktischen Operationen und in der Strahlentherapie nachteilig ist;
   - der Kopf ist nicht ausreichend fixierbar; besonders in seitlicher Richtung (nach lateral) ist die Kopfbeweglichkeit zu wenig einschränkbar.
b) Fixation des Kopfes mit Gesichtsmaske:
   Der Kopf liegt in Rückenlage in einer vorgeformten Schale und das Gesicht wird von einer vorher individuell angefertigten Maske überdeckt, welche lateral verankert ist. Auf Grund der Hautverschiebungen auf den Gesichtsknochen ergibt sich jedoch ein geringes Bewegungsspiel zwischen Gesicht und Maske, so daß eine hochgenaue Fixation des Kopfes nicht erzielbar ist, wobei zudem noch folgende Punkte negativ sind:
   - Das Operationsgebiet im Gesichtsbereich ist nicht zugänglich, da dieser weitgehend verdeckt ist;
   - Anschwellung der Haut unter der straff sitzenden Maske;
   - die völlige Abdeckung des Gesichtes ist patientenbelastend;
   - hoher Kosten- und Zeitaufwand.
c) Fixation des Kopfes durch Einklemmung:
   Der Kopf des Patienten wird durch Stellschrauben von mehreren Seiten her in einen Metallring eingeklemmt. Nachteile sind hierbei:
   - Die Operationsgebiete Gesicht und Hinterkopf sind weitgehend verdeckt und damit nicht oder nur schwer zugänglich;
   - hohe Kosten des Metallringes;
   - verschiedene Lagerungen des Patienten für unterschiedliche Eingriffe sind nur sehr bedingt möglich;
   - häufig auftretende Hautverschiebungen.
d) Fixation des Kopfes durch Verschraubung des Schädels:
   Der Kopf des Patienten wird an mehreren Stellen über einen Metallring verschraubt. Nachteilig ist dabei folgendes:
   - Die Verschraubung am Schädelknochen stellt eine invasive Methode dar und ist somit nur bei bestimmten Indikationen möglich und gerechtfertigt;
   - die psychische Belastung des Patienten ist erheblich;
   - die Methode ist nur eingeschränkt auf bestimmte Lagerungen des Patienten anwendbar.
e) Fixation des Kopfes über die Zähne (sogenannte Gill-Thomas-Cosman-Halterung gemäß GB-A-2 213 066):
   Ein Abdruck der Oberkieferzähne wird hierbei in den Mund eingesetzt (zementiert) und über eine Traverse an einer auf Nasenhöhe verlaufenden ringartigen Haltevorrichtung gegen eine Hinterkopfstütze verspannt. Das hat zur Folge, daß die Lage des Patientenkopfes zwangsweise d. h. unter erheblicher Kraftausübung vorgegebenen Anschlußpunkten angepaßt werden muß. Durch die erforderliche Zementierung an den Oberkieferzähnen ist diese Vorrichtung bei älteren, zahnlosen Patienten nicht anwendbar. Weiter sind folgende Punkte nachteilig:
   - Verschiedene Operationsgebiete sind auf Grund des platzraubenden Halterahmens nicht zugänglich (z.B. kann in der HNO-Chirurgie ein Endoskop nicht durch die Nase eingeführt werden);
   - die Positionierung des Patientenkopfes ist umständlich und schwierig, somit zeitaufwendig und patientenbelastend;
   - das Verspannen und Zementieren des Abdrucklöffels im Mund ist patientenbelastend, insbesondere auch beim Lösen des Abdrucks;
   - die Halterung ist aufwendig, zumal zur Fixierung des Kopfes auch Ohrenpfropfen und über die Schädeldecke verlaufende Riemen vorgesehen sind, die die möglichen Operationsregionen noch weiter einschränken;
   - die Lagerung des Patientenkopfes ist nur eingeschränkt möglich, da sich der Halterahmen bis zum Genick erstreckt, wodurch auch insbesondere seitliche Hinterkopfbereiche nicht zugänglich sind.

Aus dieser gattungsbildenden GB 2 213 066 A ist im einzelnen eine Vorrichtung zur Fixierung des menschlichen Kopfes bekannt, die an einer Haltevorrichtung eine lösbar angebrachte Oberkieferquerplatte und einen an der Oberkieferquerplatte festlegbaren Oberkiefer-Abdrucklöffel aufweist. Dabei wird der Oberkiefer-Abdrucklöffel jedoch mittels außen am Kopf verlaufender Bänder fixiert.

Die GB-A-2 164 856 offenbart eine Fixiervorrichtung, bei der der Kopf mittels Ohrenpfropfen und einer Nasenstützvorrichtung gehalten wird. Aus der US-A-4 602 622 ist es bekannt, einen Kopf durch an einem Ring vorgesehene Gewindeelemente zu halten, die am Kopf anstoßen. Die WO-A-90/13257 offenbart eine Kopffixierung unter Einsatz einer Beißvorrichtung. Ferner offenbart die US-A-5 230 623 eine Beißvorrichtung, die einen Oberkiefer-Abdruck aufweisen kann und mittels Bändern am Kopf fixierbar ist. Diese Vorrichtung dient jedoch zur Positionierung von Fixpunkten und nicht zur Fixierung des Kopfes.

Der Erfindung liegt deshalb die Aufgabe zugrunde, eine Vorrichtung und ein Verfahren zur Fixierung des menschlichen Kopfes zu schaffen, welche(-s) bei der Anbringung bzw. Entfernung des Abdrucklöffels weniger zeitaufwendig und patientenbelastend sind.

Diese Aufgabe wird gelöst mit einer Vorrichtung gemäß den Merkmalen des Anspruches 1 bzw. mit einem Verfahren gemäß Anspruch 14 bzw. 16. Weitere Lösungen bieten ein dazu geeigneter Abdrucklöffel gemäß Anspruch 17 und ein Operationssimulationsverfahren gemäß Anspruch 23.

Im Gegensatz zu den eingangs beschriebenen Vorrichtungen bzw. Verfahrensweisen erfolgt bei der erfindungsgemäßen Vorrichtung bzw. dem Verfahren die Fixierung des menschlichen Kopfes im wesentlichen durch Unterdruckkräfte, die zwischen dem zu fixierenden Kopf und einem individuell der jeweiligen Person abgenommenen Abdruck des Oberkiefers bzw. des sogenannten Harten Gaumens (palatinum durum) und Zähnen, sofern vorhanden, wirken. Das Lösen des Abdrucks von dem Oberkiefer bzw. Hartem Gaumen des Patienten nach Abschluß der Untersuchung bzw. der Behandlung ist durch Abschalten des Unterdrucks mit sehr geringem Kraftaufwand und damit sehr patientenschonend möglich. Die Fixierung bzw. Befestigung des Oberkiefer-Abdrucklöffels erfolgt ebenso in besonders einfacher, reproduzierbarer und patientenschonender Weise durch Anlegen von Unterdruck.

Durch diese Fixierung mittels Unterdruck kann dabei sichergestellt werden, daß insbesondere auch bei zahnlosen Patienten (z.B. Unfallopfer) und selbst nach mehrfacher Benutzung eines Abdrucks immer eine feste, exakt gleichbleibende Verbindung durch Unterdruck ermöglicht wird. Als Mittel zur Erzeugung von Unterdruck sind einfache Vakuumpumpen mit einstellbarer und zur Überwachung der sicheren Abdruckfixierung kontrollierbarer Saugdruckhöhe, Schlauchverbindungen und Absperrventile zu verwenden. Zur Unterstützung der Vakuum-Wirkung können durch Abtragung von Abdruckmasse am an sich fertigen Abdruck flache Hohlräume oder Unterdruckkanäle erzeugt oder bevorzugt bereits beim Abdruck eingeformt werden, sowie ein Abschlußstutzen im vorderen Oberkieferbereich des Abdrucks und des Oberkiefer-Abdrucklöffels eingesetzt werden. Zudem ist dieses System im Sterilisator einfach zu reinigen und die Betriebskosten sind gering, da für jeden einzelnen Patienten nur ein Abdruck aus preiswerten Abdruckmassen verwendet wird, während die übrigen Bauteile der Fixierhalterung immer wieder verwendbar sind.

Durch die beliebig auf der Grundplatte positionierbaren stativ- oder säulenartigen Abstützelemente bzw. Basishalter mit mehreren Schwenkachsen oder Kugelköpfen bleibt dabei der Patientenkopf für den behandelnden Arzt nahezu vollständig zugänglich für jegliche Art von Eingriffen. Wesentlich ist hierbei die schlanke, stabförmige Ausführung der Abstützelemente, die jedoch ausreichend stabil sind und im Gegensatz zu den bekannten, massiven Metallringen an beliebiger Stelle auf der Grundplatte so positioniert werden können, daß sie beim geplanten Eingriff nicht stören und in allen Koordinatenachsen bzw. Freiheitsgraden eingestellt werden können.

Von besonderem Vorteil ist dabei eine ähnlich aufgebaute und damit variabel positionierbare Gegenfixierung zur zusätzlichen Sicherung des Patientenkopfes, vor allem in Regionen des Ober- und Hinterkopfbereiches oder im Schulterbereich, wodurch die Fixierung des Kopfes insbesondere bei unruhig/wachen Patienten oder bei größeren Krafteinwirkungen, wie bei Knochen- oder Knorpelbearbeitungen, auf den Patientenkopf gesichert wird. Zudem ermöglicht dies eine gleichmäßige Druckverteilung und Entlastung des Patienten. Insbesondere die Schulterfixierung ist hierbei von eigenständiger Bedeutung, da hierdurch selbst ohne Unterdruck eine Fixierung des Patientenkopfes durch Ausübung eines Gegendrucks zum Oberkieferabdruck mit Streckung des Halsbereiches erreicht werden kann. Dieses "Streck"-Verfahren eignet sich somit insbesondere für Eingriffe im Halsbereich.

Besonders vorteilhaft ist zudem die Anbringung von passiven oder aktiven Eichpunkten, die in der modernen Medizin Fixpunkte und Bezugsebenen bei der praktischen Anwendung von bildgebenden Verfahren, wie CT (Computertomographie) oder MRI (Kernspintomographie), festlegen. Die beanspruchte Fixiereinrichtung erlaubt nämlich nach Erstellung eines Abdrucks und Anbringung eines Eichgestänges am Oberkiefer-Abdrucklöffel oder an der damit fest verbundenen Oberkieferquerplatte Eichpunkte zu setzen, die mit oder selbst ohne weitere Fixierung des Patientenkopfes beim bildgebenden Verfahren (CT oder MRI) dann als Bezugspunkte für weitere Maßnahmen, wie die Eichung des Patienten zur Operationsvorbereitung oder zur Operationssimulation, verwendet werden können. Dies ermöglicht eine neuartige, effiziente Operationsvorbereitung mit einer Simulation des geplanten Eingriffs, da die Operationsinstrumente noch ohne aufgelegten Patienten voreingestellt werden können und dabei anhand der computergenerierten, virtuellen Patientenbilder z. B. die Einführungsrichtung und -länge von Instrumenten unter Umgehung lebenswichtiger, auf den computergenerierten Bildern erkennbaren Nerven- oder Blutbahnen festgelegt werden. Nach Auflegen des Patienten und Korrelation der Computerdaten mit den Eich- bzw. Bezugspunkten kann dann die Operation relativ rasch durchgeführt werden.

Im folgenden werden bevorzugte Ausführungsbeispiele anhand der Zeichnungen näher beschrieben und erläutert. Hierbei zeigen:
- Fig. 1: eine Vorrichtung zur Fixierung eines Patientenkopfes mit mehreren Abstützelementen in Draufsicht;
- Fig. 2: die Vorrichtung gemäß Fig. 1 in Seitenansicht mit schematisch dargestelltem Patientenkopf;
- Fig. 3: die Vorrichtung gemäß Fig. 1 und 2 in Vorderansicht mit Blickrichtung vom Körper zum Kopf eines nicht dargestellten Patienten;
- Fig. 4: eine bevorzugt angeschlossene Halterung für Eichpunkte in Draufsicht;
- Fig. 5: die Halterung für Eichpunkte in Seitenansicht mit Blickrichtung gemäß Pfeil A in Fig. 4;
- Fig. 6: eine Seitenansicht ähnlich Fig. 2, jedoch von der gegenüberliegenden Seite, mit einer Grundplatte mit aufgesetzter Fixiervorrichtung und ähnlich gestalteter Gegenfixierung;
- Fig. 7: eine Fixiervorrichtung ähnlich wie in Fig. 3, jedoch in Blickrichtung vom Kopf zum Körper des Patienten;
- Fig. 8: eine Oberkieferquerplatte in Draufsicht in der Körper-Längsachse mit daran befestigten Haltestäben zur Fixierung an einem Abstützelement;
- Fig. 9: ein Abstützelement in Stativform mit Basishalter, einem Grundstab und einem Haltestab mit zentral arretierbaren Gelenkverbindung in Seitenansicht;
- Fig. 10: ein Abstützelement gemäß Fig. 9 mit einer zentralen Feststellschraube in Vorderansicht;
- Fig. 11: ein Abstützelement in Stativform gemäß Fig. 9 und 10 mit Basishalter, teleskopierbaren Halte- und Grundstäben und Kugelgelenken mit zentraler Arretiervorrichtung in Draufsicht;
- Fig. 12: eine Halterung für Eichpunkte ähnlich Fig. 4 und 5 mit Schraubfixierung, Querdistanzstab und einem Lateraldistanzstab;
- Fig. 13: einen Oberkiefer-Abdrucklöffel in Perspektivansicht;
- Fig. 14: die Grundplatte gemäß Fig. 1 im Grundriß mit Erweiterungsplatten in Modulbauweise ; und
- Fig. 15: eine bevorzugte Gegenfixierung für den Schulterbereich in Seitenansicht.

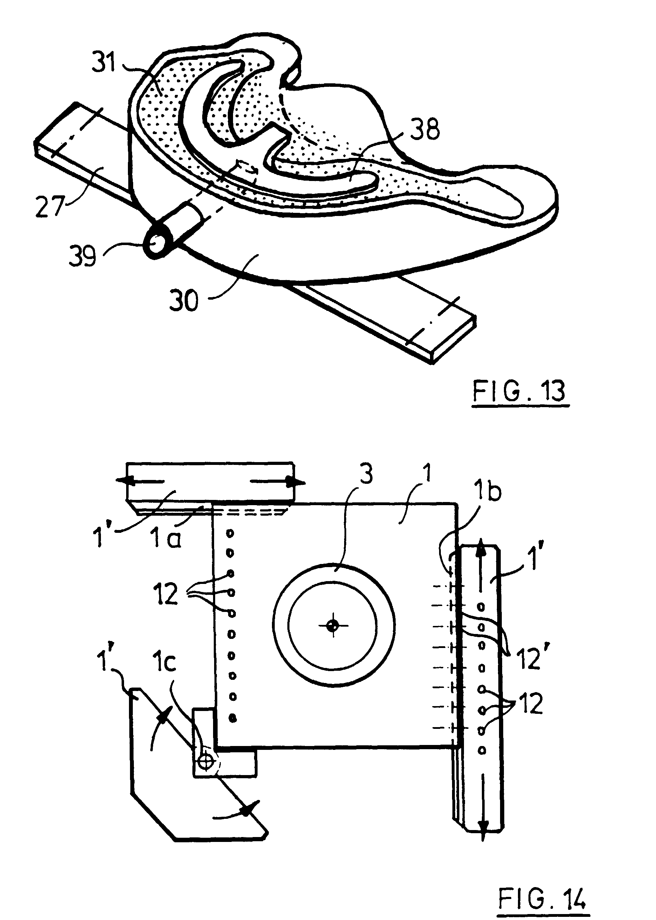

Eine aus magnetisierbarem Stahl gefertigte Grundplatte 1, an die in Modulbauweise weitere Plattenteile 1' (vgl. Fig. 14) angeschlossen werden können, ist an ihrer Unterseite mit stabilen, hier nur schematisch angedeuteten Anschlüssen 2, beispielsweise Verschraubungen, mit einem Operationstisch verbunden. Die Grundplatte 1 kann an diesen Anschlüssen 2 gegenüber dem OP-Tisch in horizontaler und in vertikaler Richtung verstellt und bis zu einem gewissen Grad gedreht werden, wobei in jeder Lage ein hohes Maß an Festigkeit gewährleistet werden kann. Auf der als Bezugsbasis dienenden Grundplatte 1 liegt zentral ein Kopfauflagering 3 (oder eine der Kopfform angepaßte Kopfauflage) und ist je nach Lagerung des Patienten frei verschiebbar. Bei Rücklage des Kopfes besteht der Kopfauflagering 3 aus einem gering kompressiblen Hartgummiring, auf den der Hinterkopf gebettet wird. Dabei stehen für die verschiedenen Kopfgrößen und Operationspositionen Kopfauflageringe 3 unterschiedlichen Durchmessers und unterschiedlicher Dicke zur Verfügung. Der Kopfauflagering 3 bzw. die geformte Kopfauflage bei Seitenlagerung des Patienten sind für die gleichmäßige Druckverteilung und Kraftübertragung vom Kopf zur Grundplatte 1 zweckmäßig. Sie vergrößern die Auflagefläche, was eine Verminderung des Flächendruckes zur Folge hat. Die vorgeformte Kopf auf läge kommt hauptsächlich bei Seitenlage des Patienten, bzw. der Untersuchungsperson in Betracht.

Zur Fixierung des Patientenkopfes sind hier etwa im Eckbereich der Grundplatte 1 mehrere frei positionierbare, stativförmige Abstützelemente 4 als kraftübertragende Teile der Kopfhalterungs-Vorrichtung vorgesehen. Diese bestehen jeweils aus einem Basishalter 5, 15 und einem damit fest verbundenen, bevorzugt eingeschraubten Grundstab 7, 17 und einem mit diesem gelenkig verbundenen Haltestab 8, 18, wobei das Bezugszeichen mit vorangestellter Ziffer "1" jeweils das gleiche Bauelement auf der gegenüberliegenden Längsmittelebene der Grundplatte 1 bezeichnet. Der Haltestab 8 bzw. 18 weist an seinem Ende jeweils eine Befestigungsstelle 28 mit Schraubarretierung 10 für je ein Ende einer mittigen Oberkieferquerplatte 27 auf.

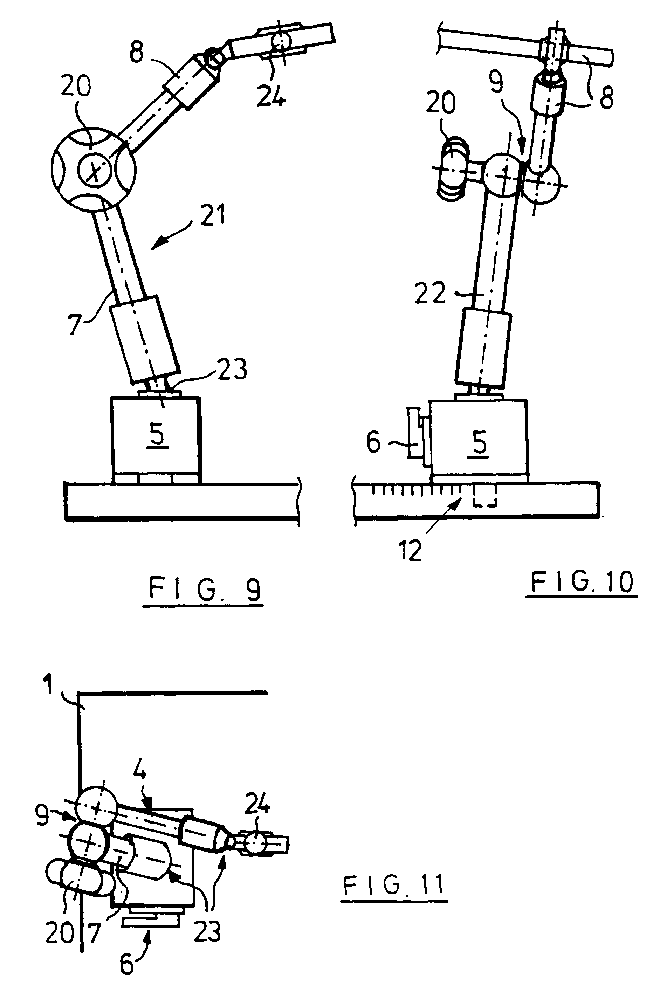

Der Basishalter 5, 15 besitzt einen Ein-/Aus-Schalter 6, 16 für im Basishalter eingebaute starke Dauermagnete, womit er an jeder Stelle der magnetisierbaren Grundplatte 1 magnetisch unverrückbar verankert (bzw. gelöst) werden kann. Bei einer alternativen pneumatischen Ausführung bezeichnet 6, 16 ein Betätigungsventil zur Aktivierung von Vakuum zum Festsaugen der Basishalter 5, 15 auf der Grundplatte 1. Diese braucht bei dieser Ausführung nicht magnetisierbar zu sein, so daß dann eine einfache Kunststoffplatte verwendet werden kann, wodurch zudem auch ein Einsatz beim MRI-Scanner einfach möglich ist. Die Basishalter 5, 15 können jedoch auch in entsprechende Bohrungen 12 in der Grundplatte 1 eingesteckt werden (vgl. Fig. 10).

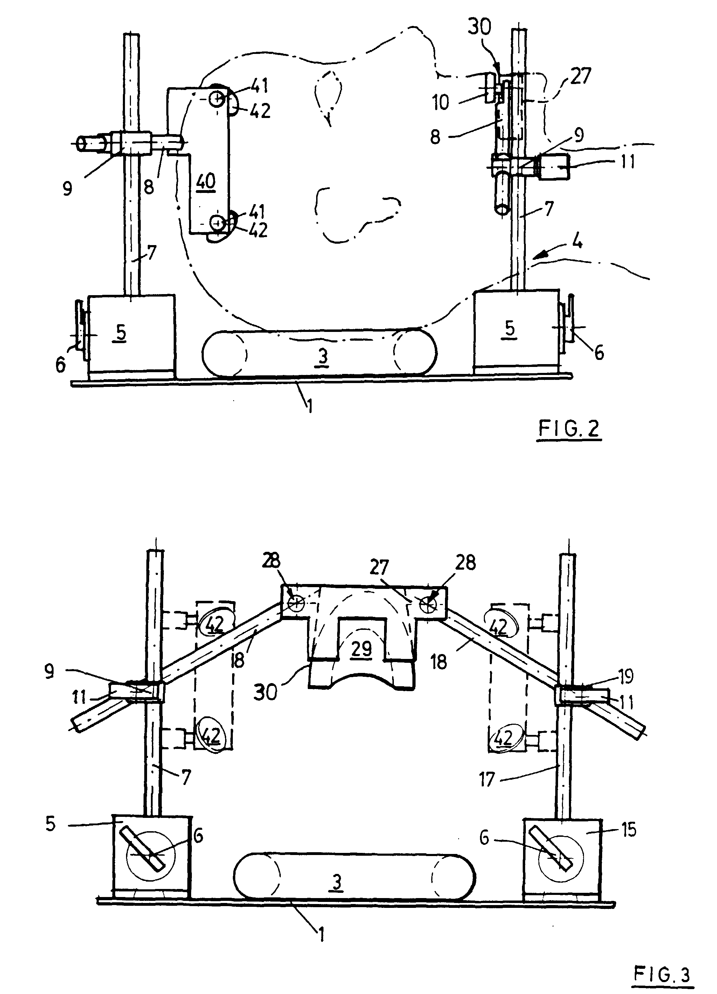

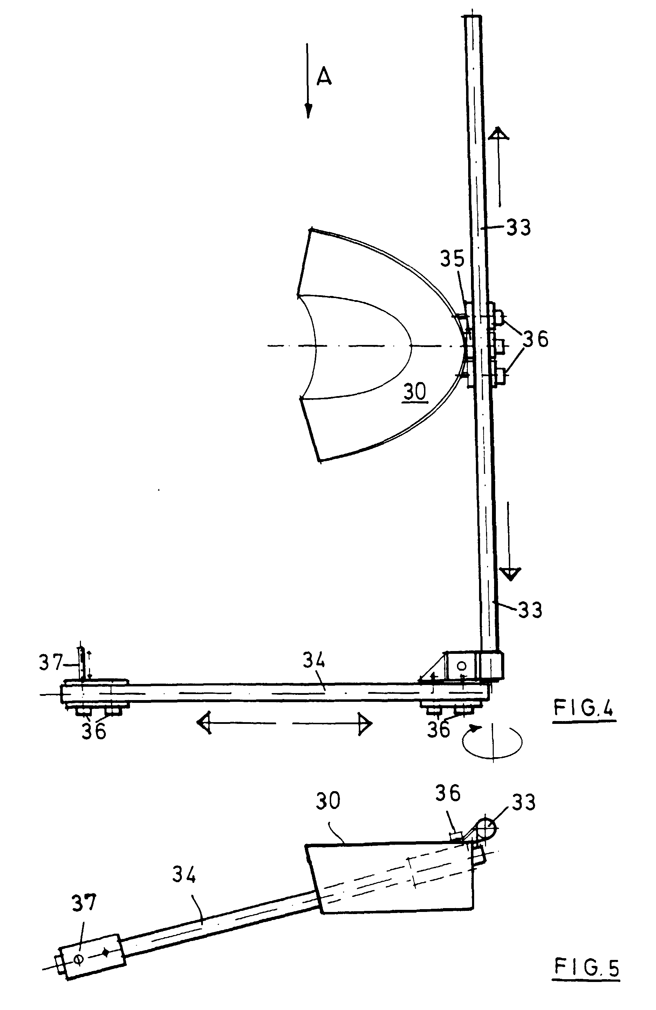

Die jeweilige Gelenkverbindung 9, 19 zwischen Grundstab 7, 17 und Haltestab 8, 18 wird mittels einer zentralen Schraubenarretierung 11 fixiert und sichergestellt. Bei der abgewandelten Ausführung in Stativform, jedoch mit gleichem Basishalter 5, 15 gemäß Fig. 9 bis 11, wird die Kraftabstützung des Patientenkopfes durch teleskopierbare Halterohre 22 als Grund- und Haltestäbe 7, 8 und Kugelgelenke 23 als Gelenkverbindung 9 bewirkt. Das Ende des rechten bzw. linken Haltestabes 8, 18 bildet je eine Anschlußstelle 28 zur Befestigung an der Oberkieferquerplatte 27. Diese Oberkieferquerplatte 27 wird mit einem mit einer Abdruckmasse 31 (vgl. Fig. 4 und 13) gefüllten Oberkiefer-Abdrucklöffel 30 mittels einer Verbindung, insbesondere eines schnellhärtenden Klebers, an dessen Flachseite verbunden. Die Enden der Oberkieferquerplatte 27 (vgl. Fig. 3) verlassen dabei den Mundraum des Patienten etwa im Bereich der Mundwinkel, können jedoch auch zur Nase oder zum Kinn hin geführt sein. Die Oberkieferquerplatte 27 kann dabei auch einseitig als Kragarm gelagert sein.

Die Oberkieferquerplatte 27 besteht z. B. aus rostfreiem ChromStahl und weist etwa 2 mm Dicke auf. Ihre Größe und Form ist variabel und kann damit den jeweiligen operativen Anforderungen gut angepaßt werden, z. B. auch aus hochfesten Karbonfasern bestehen. Im mittigen Bereich 29 der querverlaufenden Oberkieferquerplatte 27 ist ein U-förmiger Raum ausgespart, um z. B. einen Tubus einzuführen oder für Eingriffe Platz und Übersicht im Mundraum zu gewinnen. Die Oberkieferquerplatten-Enden an den Befestigungsstellen 28 sind, ebenso wie die gesamte Oberkieferquerplatte 27, nach Größe und Form, vor allem in ihrer Ausladung variabel den Bedürfnissen der jeweiligen Vorgehensweise bei dem Behandlungseingriff angepaßt.

In Fig. 2 und 3 ist die Vorrichtung zur Fixierung eines Patientenkopfes in Operationsposition dargestellt. Hieraus ist ersichtlich, daß die Abstützelemente 4 vom Krafteinleitungspunkt am Oberkiefer-Abdrucklöffel 30 portal- oder brückenartig zur Grundplatte 1 verlaufen und dabei nach Arretierung der Gelenke 9 bzw. 23 die Kraftübertragung in allen Richtungen in stabiler Weise übernehmen. Dabei liegt jedoch insgesamt eine schlanke Bauform vor, so daß der Operations- oder Behandlungsbereich am Kopf für den Arzt sehr gut zugänglich ist.

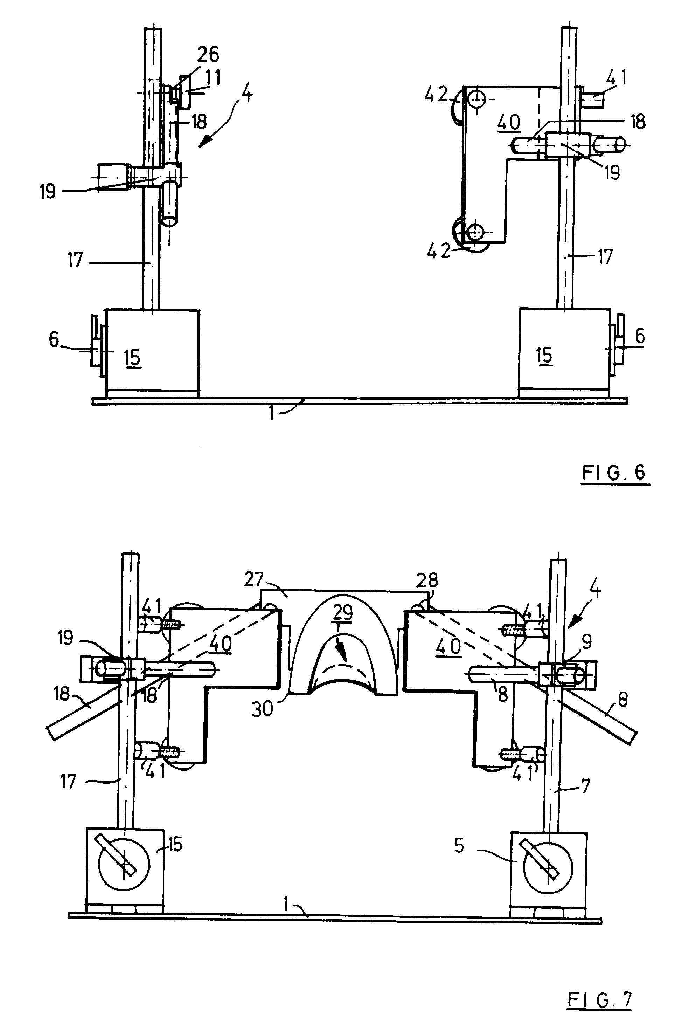

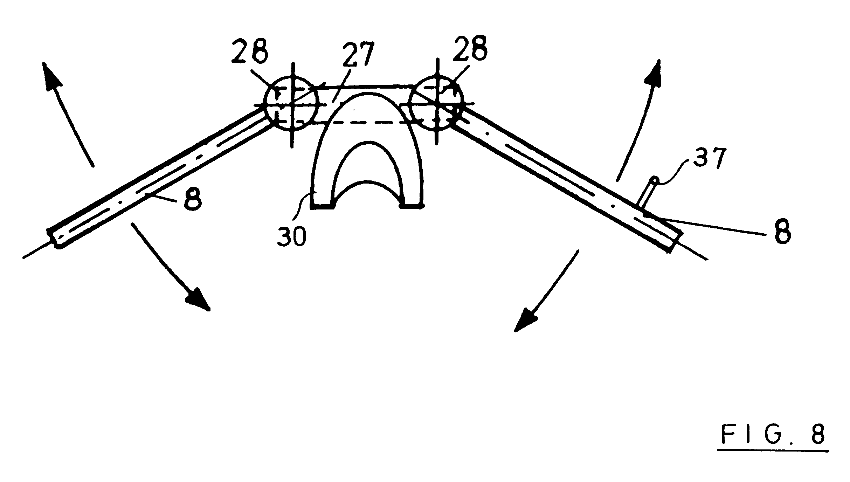

Bei stereotaktischen Operationen sind zudem Bezugspunkte wesentlich, wobei der vorstehend beschriebene Aufbau der Fixiervorrichtung bzw. Elemente davon auch als Halterung für Eichpunkte 37 (vgl. Fig. 4, 5, 8 und 12) dienen kann. Dieses sog. Eichgestänge besteht aus einem Querdistanzstab 33, der mit dem Oberkiefer-Abdrucklöffel 30 dreh- und/oder verschiebefest verbunden ist. Ein Lateraldistanzstab 34 ist an den Querdistanzstab 33 durch eine Schraub- oder Klemmverbindung 35, 36 einstell- und fixierbar befestigt. Als Eichpunkte 37 dienende Markerstifte können entweder selbst die Eichpunkte bilden oder als Träger für Marker (z.B. in Form von Bleikügelchen) dienen. Als Referenz- oder Eichpunkte können jedoch andere Sensorentypen verwendet werden z. B. optische, radioaktive oder induktive Sender. Die Markerstifte können auch an jeder anderen Stelle der Halterung, z. B. am Querdistanzstab 33 oder an einem der Haltestäbe 8, 18 (vgl. Fig. 8) angebracht werden.

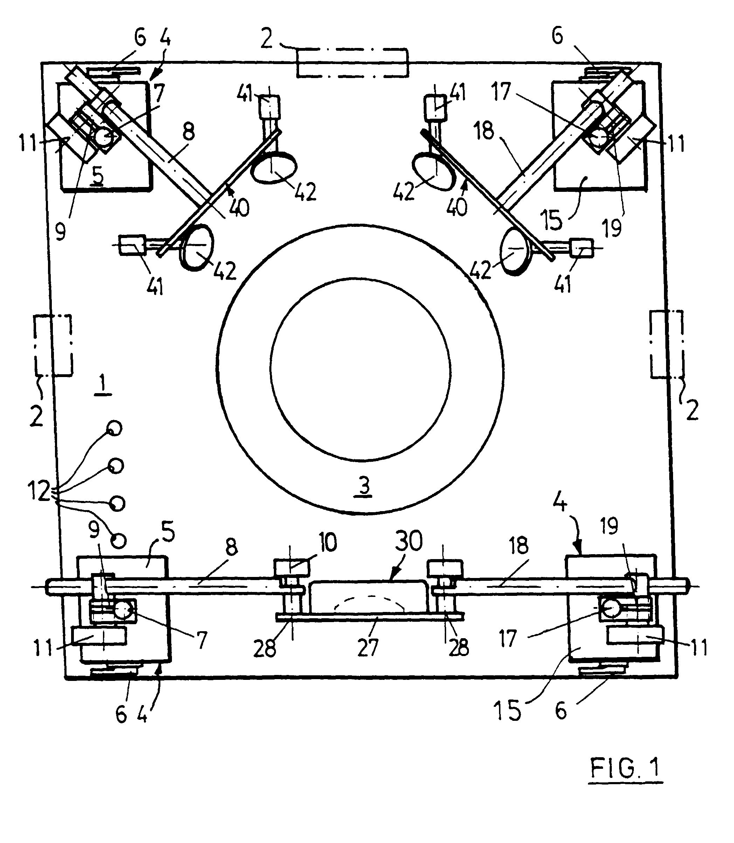

In Fig. 1 bis 3 sowie Fig. 6 und 7 ist eine Gegenfixierung 40 für den Hinterkopf des Patienten dargestellt, wie sie insbesondere bei Operationen mit starker Kraftausübung auf den Schädel (z. B. Meißeln, Fräsen) zweckmäßig ist. Bei ansonsten gleichem Aufbau mit Basishaltern 5, 15, Grundstäben 7, 17 und Haltestäben 8, 18 usw. weist die Gegenfixierung 40 mehrere Stellschrauben 41 und daran befestigte Gegendruckscheiben 42 auf. Diese Gegenfixierung 40 ist jedoch für kraftfreie Eingriffe wie Strahlenbehandlungen nicht unbedingt erforderlich, so daß der Patientenkopf allein an dem/den mit dem Oberkiefer-Abdrucklöffel 30 verbundenen Abstützelement(-en) 4 gehalten ist.

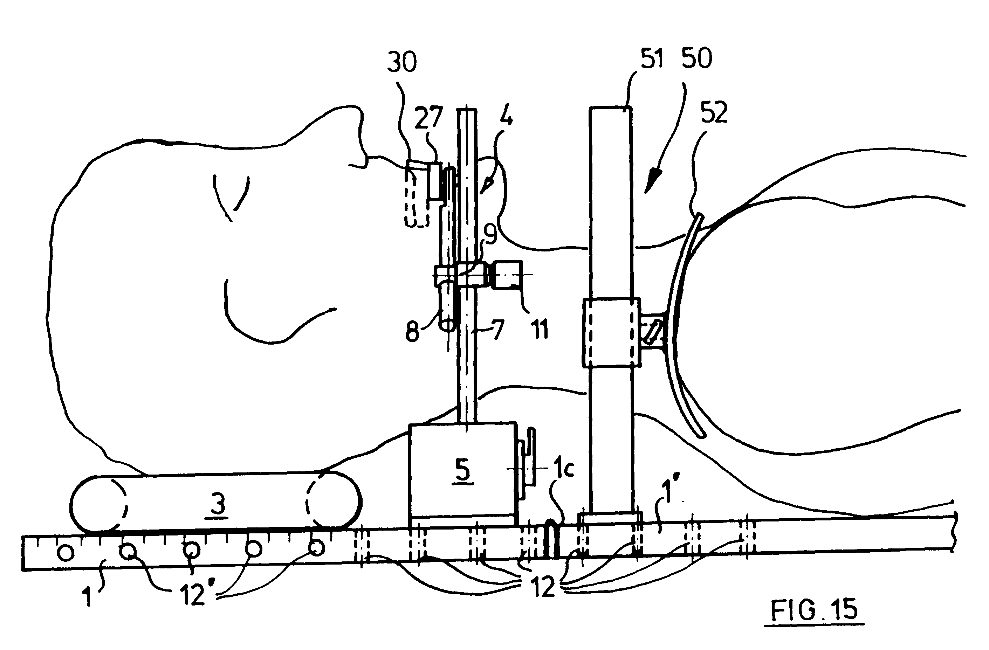

In Fig. 15 ist als alternative Gegenfixierung 50 ein höhenverstellbarer Schultersattel 52 vorgesehen, der eine exakte Repositionierung erlaubt. Der Schultersattel 52 ist dabei an einem an der Grundplatte 1 mittels Steckverbindung 12 verankerten Grundstab 51 verschiebbar und drehbar gelagert, so daß nach Einstellung auf die Patientenschultern eine Gegenkraft zu der Oberkieferfixierung aufgebracht werden kann und hierbei die Wirbelsäule im Halsbereich gestreckt wird. Diese Gegenfixierung 50 ist somit insbesondere für Eingriffe im Halsbereich zweckmäßig. Weitere Gegenfixierungen 50 können auch am Unterkiefer in beliebiger Stellung positioniert werden.

In Fig. 8 ist der Oberkiefer-Abdrucklöffel 30 zusammen mit angeschlossenen Haltestäben 8 dargestellt. An diesen kann ein Eichpunkt 37 angebracht sein, so daß der Patient mit der hier dargestellten Einheit gescannt werden kann, wobei diese Einheit allein durch den an den Oberkiefer-Abdrucklöffel 30 angelegten Unterdruck exakt und repositionierbar gehalten wird. Anschließend kann dann der Patientenkopf durch Verbindung der Haltestäbe 8 mit den Abstützelementen 4 zur Operation festgestellt werden, z.B. durch Einsetzen in ein in den nachfolgenden Figuren 9 bis 11 dargestelltes Lagerauge 24, Einschalten der Basishalter 5 und Arretieren der Gelenke 9 bzw. Kugelgelenke 23.

In Fig. 9 bis 11 sind Ansichten der bevorzugten Ausführung des Abstützelementes 4 dargestellt, nämlich in der aus der Feinwerktechnik an sich bekannten Stativform, wobei diese Stative 21 durch eine zentrale Arretiervorrichtung 20 "verriegelbar" oder in einer Position starr feststellbar sind. Hierbei drückt durch Betätigung der Arretiervorrichtung 20 ein Kolben auf nicht dargestellte Spannzangen in den Kugelgelenken 23 und fixiert diese. Die Spannung erfolgt dabei unter Fluiddruck ( insbesondere Hydraulik) in den hohlen Halterohren 22, so daß alle Gelenke 9 bzw. 23 je eines Abstützelementes 4 simultan arretiert oder starr gemacht werden können.

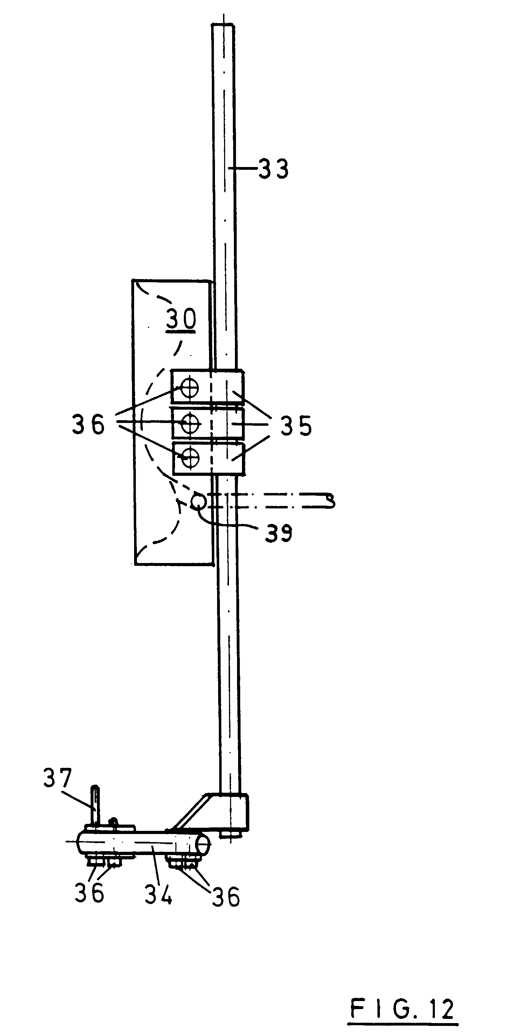

In Fig. 13 ist zur Verdeutlichung eine Perspektivansicht des Oberkiefer-Abdrucklöffels 30 mit eingefüllter Abdruckmasse 31 (punktiert angedeutet) dargestellt. Bei diesem "Vakuum-Kit" wird zur Schaffung von Unterdruckkanälen (oder -taschen) für die spätere Vakuumanlegung wenigstens ein flexibler Streifen 38, der bevorzugt aus einer dünnen Gummimatte U- oder sternförmig ausgestanzt ist, vor dem Abdruck des Oberkiefers bzw. Gaumens in die Abdruckmasse 31 eingelegt. Hierbei ist zudem auch ein Anschlußstutzen 39 ausgebildet, so daß nach Abnahme des Kieferabdrucks und Aushärten der Abdruckmasse 31 die Streifen 38 mehrteilig oder eintelig abgezogen werden können. Dadurch werden im für jeden Patienten individuellen Abdruck flache Taschen oder Kanäle ausgebildet, die bei Vakuumanlegung (vgl. Fig. 12) an den Oberkiefer-Abdrucklöffel 30 über den Anschlußstutzen 39 Unterdruckkammern zum Oberkiefer/Gaumen hin ausbilden. Durch Überwachung des Unterdrucks (etwa 0,2 atm) kann zudem der exakte Sitz des Abdrucklöffels 30 und damit die genaue Positionerung kontrolliert werden. Hierbei können auch standardisierte Abdrucklöffel 30 mit mehreren vorfabrizierten Größen Anwendung finden.

Von wesentlicher Bedeutung ist weiterhin die in Fig. 14 dargestellte modulartige Erweiterbarkeit der Grundplatte 1 durch mittels Quer-/Längs- oder Drehführungen la, 1b und lc angeschlossene weitere Grundplatten 1'. Diese Grundplatten 1' können dabei ebenso wie die Grundplatte 1 eine Vielzahl von rasterförmig angeordneten Steckbohrungen 12 (und/oder an den Seitenkanten 12') aufweisen, in die die Basishalter 5, 15, die Grundstäbe 7, 17 (oder 51 im Falle der Schulter-Gegenfixierung) einsteckbar sind. Dabei kann dann die Grundplatte 1' im Schulterbereich zusammen mit der Gegenfixierung 50 bezüglich der Kopflängsmittelebene um die Drehführung lc verschwenkt werden, um dem Operateur z. B. einen besseren Zugriff auf einen bestimmten Halswirbel zu ermöglichen. Es sei angemerkt, daß dabei auf die ursprüngliche stereotaktische Positionierung, z. B. mittels der Markerstifte 37, zurückgegriffen werden kann, da die Schwenkbewegung (oder auch eine translatorische Relativbewegung) zwischen den Grundplatten 1 und 1' durch Skalen oder Winkel-/Längenmeßgeber an den Führungen la, 1b oder 1c in allen Kordinatenachsen oder Freiheitsgraden erfaßt wird, so daß der Patient auch umgelagert werden kann.

Im folgenden soll die praktische Anwendung der erfindungsgemäßen Vorrichtung zusammen mit den in Fig. 4, 5, 8 bzw. 12 dargestellten Eichpunkten 37 anhand der CAS (computer assisted surgery) dargestellt werden: Die CAS stellt eine interoperative Navigationshilfe dar. Vor der Operation wird der Patient im CT oder im MRI gescannt. Anschließend werden die einzelnen Schnitte zu einem 3D-Objekt mit entsprechenden stereotaktischen Raumkoordinaten am Bearbeitungscomputer rekonstruiert (generiert) und auf ein Display oder einen Monitor im Operationssaal transferiert. Dieses virtuelle Bild wird im Operationssaal auf den Patienten mit Hilfe eines mit dem Computer gekoppelten, passiven mechanischen Armes (z. B. ähnlich gemäß Fig. 12), an dessen Ende sich eine Sonde befindet, geeicht. Sensoren an jeder der sechs Rotationsachsen des Armes teilen dem Computer die relativen Winkel der Armteile zueinander mit, wodurch der Computer in der Lage ist, die stereotaktischen Koordinaten der Sondenspitze zu errechnen. Durch Anfahren mehrerer Punkte (z. B. anatomische Punkte oder durch röntgendichte Eichpunkte, -sog. Marker- gekennzeichnete Referenzpunkte) am Patienten bzw. an der Eichvorrichtung und entsprechende Korrelation zum rekonstruierten 3D-Objekt am Bildschirm ist es dem Computer möglich, dieses 3D-Objekt in diesen virtuellen Raum einzupassen. Je besser die angefahrenen Punkte mit dem am Bildschirm korrelierten Punkten übereinstimmen, desto genauer stimmen virtuelles Bild und Patientenkopf überein, was zu einer hohen Genauigkeit des Systems führt. Der Operateur kann sich während der Operation mit Hilfe von rekonstruiertem 3D-Objekt und mehreren zweidimensionalen Bildern, die immer die Sondenspitze anzeigen, orientieren.

Die erfolgreiche Anwendung dieser Methode ist nur dann gewährleistet, wenn eine exakte Fixierung des Patientenkopfes und damit Eichung gegeben ist. Wie eingangs beschrieben, sind bekannte Systeme derzeit nicht imstande, Bewegungen des Patienten exakt zu registrieren. Selbst bei einem System mit aktiver Kopfbewegung-Registrierung ist eine Fixation zur Ansteuerung von Zieleinrichtungen unumgänglich. Deshalb ist derzeit bei bekannten Fixationen vor jeder Verwendung der Sonde eine Kontrolle der Kopfposition durch Anfahren von entsprechenden Eichpunkten (landmarks) notwendig. Bei jeder Kopfbewegung ist daher eine Nacheichung erforderlich. Die Folgen sind u. a. erhebliche Verlängerungen der Operationszeit. Außerdem kann eine Lageveränderung zwischen Eichkontrolle und Anwendung der Sonde für Planung, Orientierung und Navigation schwerwiegende Folgen für den Patienten mit sich bringen, weil dieser Fehler vom System nicht erfaßt wird und somit eine Fehlinterpretation die Folge sein kann. Somit ist die Verläßlichkeit des gesamten Systems eingeschränkt. Für die Eichung bedarf es eines ausreichend großen und gut zugänglichen Hautareals. Dies ist notwendig für die Markereichung, bei welcher mehrere Marker auf der Haut angebracht werden. Zur Verbesserung der Markereichung dient die Oberflächeneichung, bei der die zur Nacheichung dienenden Hautareale durch das Anbringen der Halterung nicht verschoben werden dürfen. Durch das Verschieben der Haut und/oder der auf ihr angebrachten Marker in Relation zum Kopf, ist eine Korrelation des realen Patientenkopfes auf das aus den CT-Daten rekonstruierte 3D-Objekt mit der erwünschten und erforderlichen Präzision nicht durchführbar. Dies gilt sowohl für die Marker- als auch die Oberflächeneichung. Bei all diesen bekannten Verfahren bestand demnach eine unbefriedigende Situation.

Bei der Anwendung der erfindungsgemäßen Vorrichtung bzw. Verfahren wird im Gegensatz dazu wie folgt vorgegangen: Zunächst wird vom Patienten ein Oberkiefer-Abdruck mittels des Oberkiefer-Abdrucklöffel 30 und der darin eingefüllten Abdruckmasse 31 vorgenommen. Erfolgt dies zeitlich kurz vor dem CT-Scan, so kann nach Einstellung der bevorzugt aus Karbonfiber bestehenden Eichvorrichtung 33 bis 37 der Patient ohne Herausnehmen des nur mit Unterdruck gehaltenen Abdrucklöffels 30 und ohne Anwendung der zusätzlichen Kopfhalterung im CT gescannt werden. Dabei wird die Eichvorrichtung 33 bis 37 durch Verschieben/Verschwenken der Distanzstäbe 33, 34 so eingestellt, daß sich der auf der Schraubhalterung 36 befindliche Markerstift 37 (in der Regel verwendet man Kügelchen aus röntgendichtem Material) im gescannten Bereich befinden. Dabei ist kein aktives Halten durch den Patienten erforderlich und somit der Mundraum für eine Intubation zugänglich.

Eine derartige reversible Kopffixierung in der Eingriffsposition ist insbesondere mit CT-Scans sinnvoll für Weichteil-Eingriffe, da sich diese in Abhängigkeit von der jeweiligen Lage verschieben können. Es ist jedoch oft zweckmäßig, den Patienten (und damit die Eichvorrichtung 33 bis 37) in der in Fig. 1 bis 3 dargestellten Kopfabstützung fixiert zu scannen. Ein positiver Nebeneffekt dieser exakten Kopffixierung während des CT-Scans ist die Vermeidung oftmals auftretender Bewegungsartefakte. Nach der CT-Untersuchung wird das Abstützelement 4 und der Unterdruck gelöst und der Abdrucklöffel 30 (zusammen mit der Eichvorrichtung 33 - 37) entnommen. Dieser kann dann für eine spätere Operation archiviert werden.

Dabei wird dann folgendermaßen vorgegangen: Nach Einbringung des Abdrucklöffels 30 in den Mundraum wird dieser mit der Querplatte 27, welche mit den bereits beschriebenen Abstützelementen 4 der Kopfhalterung noch in beweglichem Kontakt steht, verbunden. Anschließend wird der den patientenindividuellen Abdruck tragende oder vorfabrizierte Abdrucklöffel 30 vorsichtig auf die Zähne bzw. auf den Harten Gaumen angedrückt und mittels Unterdruck angesaugt. Anschließend, nachdem der Bediener sich durch Unterdruckkontrolle von der richtigen Lage des Abdrucklöffels 30 und damit auch der damit verbundenen Eichvorrichtung 33 bis 37 überzeugt hat, wird die Kopffixierhalterung durch Arretierung (Starrmachen) sämtlicher gelenkiger Verbindungen 9, 19 zwischen Haltestäben 8, 18 und Grundstäben 7, 17 und durch Verankerung (Aktivierung der Magnete bzw. Unterdruckquelle mittels der Schalter 6, 16) der Basishalter 5, 15 auf der Grundplatte 1 bzw. 1' fixiert.

Bei dem anschließenden operativen Eingriff, welcher nach kürzerem oder längerem Zeitraum erfolgen kann, wird nach der schon für den CT-Scan beschriebenen Vorgangsweise der Abdrucklöffel 30 in Kombination mit der Eichvorrichtung 33 bis 37 auf den Oberkiefer des Patientenkopfes eingepaßt und durch Vakuumanlegung an den Abdrucklöffel 30 sowie Arretierung der Abstützelemente 4 auf der Grundplatte 1 fixiert. Nun können die für CAS-Eingriffe erforderlichen Eichpunkte auf der Eichvorrichtung 33 bis 37 angefahren werden und somit eine Korrelation zwischen realem Patientenkopf .und dem nach dem Scan abgespeicherten, virtuellem 3D-Bild erfolgen. Besonders hervorzuheben ist die Tatsache, daß die Eichpunkte in Form der Markerstifte 37 unverrückbar in Relation zum Patientenkopf stehen. Durch Anwendung der Kopffixierhalterung in Kombination mit der Eichvorrichtung 33 bis 37 kann somit ein Maximum an Genauigkeit bei derartigen stereotaktischen Eingriffen erzielt werden.

Abschließend werden die Vorteile der erfindungsgemäßen Vorrichtung zusammengefaßt dargestellt:
- Exakte Fixation des Kopfes bei platzsparendem Aufbau;
- maximale Sicht- und Bewegungsfreiheit für den Operateur im Operationsfeld;
- Positionierung des Kopfes in variablen Lagen für den Einsatz bei diversen Eingriffen;
- kein invasiver Eingriff zur Fixierung des Kopfes erforderlich;
- keine durch die Halterungen hervorgerufenen Haut- und damit Lageverschiebungen;
- Anbringungsmöglichkeit von Instrumenten verschiedenster Art, z. B. Endoskop, an der Halterung sowie Eignung für virtuelle 3D-Endoskopie;
- Definition von exakten Eichpunkten durch die am Oberkiefer bzw. Gaumen angebrachte Eichvorrichtung, welche zugleich als Bestandteil der Fixierhalterung verwendet werden kann;
- die Bedingungen der Fixation durch die auf der Grundplatte frei beweglichen Basishalter werden den individuellen Gegebenheiten des Patientenkopfes angepaßt, und nicht umgekehrt der Patientenkopf in die Vorrichtungen eingezwängt;
- einfache und zeitsparende Handhabung durch die Unterdruckanlegung und daher besonders patientenschonend und hygienisch.

## Patentansprüche

1. Vorrichtung zur Fixierung des menschlichen Kopfes, bestehend aus:
- einer Grundplatte (1),
- mindestens einem auf der Grundplatte (1) lösbar anbringbaren Abstützelement (4) mit jeweils frei auf der Grundplatte (1) positionierbarem Basishalter (5, 15),
- einem an dem Basishalter (5, 15) befestigten Grundstab (7, 17) mit wenigstens einer Gelenkverbindung (9, 19),
- einem an der Gelenkverbindung (9, 19) befestigten, auskragenden Haltestab (8, 18), wobei am Ende des Haltestabes (8, 18) eine lösbar angebrachte Oberkieferquerplatte (27) vorgesehen ist, und
- einem an der Oberkieferquerplatte (27) anbringbaren Oberkiefer-Abdrucklöffel (30), der mittels Unterdruck am Oberkiefer fixierbar ist.

2. Vorrichtung nach Anspruch 1,
dadurch gekennzeichnet, daß
der Oberkiefer-Abdrucklöffel (30) mittels einer Schraubarretierung (10, 28) oder eines Klebers mit der Oberkieferquerplatte (27) verbindbar ist.

3. Vorrichtung nach Anspruch 1 oder 2,
dadurch gekennzeichnet, daß
an den Basishaltern (5, 15) mehrachsig schwenkbare Gelenkverbindungen (9,19) in Form von Stativen (21) mit zentraler Arretiervorrichtung (20) vorgesehen sind.

4. Vorrichtung nach einem der Ansprüche 1 bis 3,
dadurch gekennzeichnet, daß
die Grundplatte (1) an einem Operationstisch verstellbar anbringbar ist.

5. Vorrichtung nach einem der Ansprüche 1 bis 4,
dadurch gekennzeichnet, daß
die Grundplatte (1) und/oder Teile des Abstützelementes (4) einschließlich des Oberkiefer-Abdrucklöffels (30) zumindest abschnittsweise aus röntgendurchlässigem Material gebildet sind.

6. Vorrichtung nach einem der Ansprüche 1 bis 5,
dadurch gekennzeichnet, daß
die Grundplatte (1) Markierungen und/oder Steckbohrungen (12) zur Repositionierung des gelösten Basishalters (5, 15) aufweist.

7. Vorrichtung nach einem der Ansprüche 1 bis 6,
dadurch gekennzeichnet, daß
die Basishalter (5, 15) als Magnethalterungen oder als pneumatische Halterungen ausgebildet sind.

8. Vorrichtung nach einem der Ansprüche 3 bis 7,
dadurch gekennzeichnet, daß
die Gelenkverbindungen (9, 19) der Stative (21) mittels Fluiddruck arretierbar sind.

9. Vorrichtung nach einem der Ansprüche 1 bis 8,
dadurch gekennzeichnet, daß
die Grundstäbe (7, 17) und/oder Haltestäbe (8, 18) oder Stative (21) als Trag- und Aufnahmevorrichtungen für medizinische Geräte, insbesondere endoskopische Apparaturen, Wundhaken, Handauflageflächen oder stereotaktische Navigationshilfen ausgebildet sind.

10. Vorrichtung nach einem der Ansprüche 1 bis 9,
dadurch gekennzeichnet, daß
eine Gegenfixierung (40, 50) vorgesehen ist, mit der auf den menschlichen Körper über einstellbare Flächen (42, 52) ein Gegendruck zum Oberkiefer-Abdrucklöffel (30) ausübbar ist.

11. Vorrichtung nach einem der Ansprüche 1 bis 10,
dadurch gekennzeichnet, daß
auf der Grundplatte (1) ein Kopfauflagering (3) oder eine anatomisch geformte Kopfauflage vorgesehen ist.

12. Vorrichtung nach einem der Ansprüche 1 bis 11,
dadurch gekennzeichnet, daß
wenigstens ein einstellbares Eichgestänge (33, 34, 35, 36) zur Positionsfestlegung von Eichpunkten (37) vorgesehen ist, das am Oberkiefer-Abdrucklöffel (30) und/oder an der Oberkieferquerplatte (27) anbringbar ist.

13. Vorrichtung nach einem der Ansprüche 1 bis 12,
dadurch gekennzeichnet, daß
in die im Oberkiefer-Abdrucklöffel (30) eingefüllte Abdruckmasse (31) flexible Streifen (38) mit einem Unterdruck-Anschlußstutzen (39) einlegbar sind, die nach Aushärten der Abdruckmasse (31) zur Bildung von Unterdruckkanälen abziehbar ausgebildet sind.

14. Verfahren zur Fixierung des menschlichen Kopfes mittels wenigstens einem Abstützelement und einem daran befestigten, patientenangepaßten Kieferabdruck unter Verwendung einer Vorrichtung nach einem der Ansprüche 1 - 13, dadurch gekennzeichnet, daß der den Kieferabdruck tragende Abdrucklöffel (30) zunächst mittels Unterdruck am Ober- und/oder Unterkiefer angelegt und dann fixiert wird.

15. Verfahren nach Anspruch 14, dadurch gekennzeichnet, daß
der Patient lediglich mit dem durch Unterdruck angelegten Abdrucklöffel (30) und daran angeschlossenen Eichgestänge (33 - 37) durch eine bildgebende Abtastvorrichtung geführt und erst danach der Abdrucklöffel (30) mit dem Abstützelement (4) verbunden wird.

16. Verfahren zur Fixierung des menschlichen Kopfes mittels wenigstens einem Abstützelement und einem daran befestigten, patientenangepaßten Kieferabdruck unter Verwendung einer Vorrichtung nach einem der Ansprüche 1 - 13,
dadurch gekennzeichnet, daß mittels einer Gegenfixierung (40, 50) gemäß Anspruch 10 mit Flächen (42, 52) ein Gegendruck zu dem den Kieferabdruck tragenden Abdrucklöffel (30) ausübbar ist.

17. Abdrucklöffel mit einer Kiefer-Abdruckmasse, insbesondere zur Verwendung in einer Vorrichtung nach einem der Ansprüche 1 bis 13,
dadurch gekennzeichnet, daß
der Abdrucklöffel (30) lediglich mittels Unterdruck am Ober- und/oder Unterkiefer fixierbar ist.

18. Abdrucklöffel nach Anspruch 17, dadurch gekennzeichnet, daß ein Unterdruck-Anschlußstutzen (39) mit der Kiefer-Abdruckmasse (31) verbunden ist.

19. Abdrucklöffel nach Anspruch 17 oder 18, dadurch gekennzeichnet, daß der Abdrucklöffel (30) mit einer Befestigungsvorrichtung, insbesondere einer Oberkieferquerplatte (27) verbunden ist.

20. Abdrucklöffel nach einem der Ansprüche 17 bis 19, dadurch gekennzeichnet, daß in die Kiefer-Abdruckmasse (31) wenigstens ein flexibler Streifen (38) zur Bildung eines Unterdruck-Kanals in der ausgehärteten Kiefer-Abdruckmasse (31) einlegbar ist.

21. Abdrucklöffel nach einem der Ansprüche 17 bis 20, dadurch gekennzeichnet, daß einer Flachseite des Abdrucklöffels (30) ein Fixierelement für angrenzende Weichteile, insbesondere für die Zunge, zugeordnet ist.

22. Abdrucklöffel nach Anspruch 21, dadurch gekennzeichnet, daß das Fixierelement durch einen Unterdruck-Kanal gebildet ist.

23. Verfahren zur Durchführung einer Operationssimulation, dadurch gekennzeichnet, daß
der durch den Abdrucklöffel (30) nach einem der Ansprüche 17 - 22 als Fixpunkt (37) definierte Eichpunkt mit virtuellen, aus einer Patienten-Abtastvorrichtung generierten Bildern korreliert wird.

## Claims

1. Device for fixing the human head, consisting of:
- a base plate (1),
- at least one support element (4) which may be detachably mounted on the base plate (1), with respectively a base holder (5, 15) which may be positioned freely on the base plate (1),
- a base rod (7, 17) attached to the base holder (5, 15) with at least one hinged joint (9, 19),
- a projecting holder rod (8, 18) attached to the hinged joint (9, 19), there being provided on the end of the holder-rod (8, 18) a detachably mounted upper jaw cross-plate (27), and
- an upper jaw impression plate (30) which may be attached to the upper jaw cross-plate (27) and which may be fixed to the upper jaw by means of a vacuum.

2. Device according to claim 1, characterized in that the upper jaw impression tray (30) may be connected to the upper jaw cross-plate (27) by means of a screwed locking device (10, 28) or an adhesive.

3. Device according to claim 1 or 2, characterized in that on the base holders (5, 15) hinge connections (9, 19), swivellable through many axes, are provided in the form of stands (21) with a central locking device (20).

4. Device according to one of claims 1 to 3, characterized in that the base plate (1) may be attached to an operating table so as to be adjustable.

5. Device according to one of claims 1 to 4, characterized in that the base plate (1) and/or portions of the support element (4) including the upper jaw impression tray (30) are formed, at least in sections, of material through which X-rays may pass.

6. Device according to one of claims 1 to 5, characterized in that the base plate (1) has markings and/or plug-in holes (12) to re-position the detached base holder (5, 15).

7. Device according to one of claims 1 to 6, characterized in that the base holders (5, 15) are configured as magnetic mounts or as pneumatic mounts.

8. Device according to one of claims 3 to 7, characterized in that the hinge connections (9, 19) of the stands (21) may be stopped by means of fluid pressure.

9. Device according to one of claims 1 to 8, characterized in that the base rods (7, 17) and/or holder rods (8, 18) or stands (21) are configured as carrying and receiving devices for medical equipment, especially endoscopic equipment, wound clasps, hand-rest surfaces or stereotactic navigational aids.

10. Device according to one of claims 1 to 9, characterized in that a counter-fixing device (40, 50) is provided, with which a counter-pressure to the upper jaw impression tray (30) may be exercised on the human body via adjustable surfaces (42, 52).

11. Device according to one of claims 1 to 10, characterized in that on the base plate (1), a headrest ring (3) or an anatomically shaped headrest is provided.

12. Device according to one of claims 1 to 11, characterized in that at least one adjustable calibration rod (33, 34, 35, 36) is provided to establish the position of calibration points (37), which may be attached to the upper jaw impression tray (30) and/or to the upper jaw cross-plate (27).

13. Device according to one of claims 1 to 12, characterized in that flexible strips (38) may be inserted with a vacuum connection piece (39) into the impression mass (31) filled into the upper jaw impression tray (30) and these strips are formed so that they may be withdrawn after the impression mass (31) has hardened, to form vacuum channels.

14. Method of fixing the human head by means of at least one support element and one jaw impression attached thereto and adapted to the patient, by using a device according to any of claims 1 to 13,
characterized in that
the impression tray (30), carrying the impression of the jaw, is first attached to the upper and/or lower jaw and then fixed by means of a vacuum.

15. Method according to claim 14, characterized in that the patient is only guided with the impression tray (30), attached by a vacuum, and calibration rods (33 to 37) connected thereto, by an imaging scanning device and only thereafter is the impression tray (30) connected to the support element (4).

16. Method for fixing the human head by means of at least one support element and a jaw impression fastened to same and adapted to the patient, by using a device according to any of claims 1 to 13, characterized in that counter-pressure to the impression tray (30) carrying the jaw impression may be exercised with surfaces (42, 52) by means of a counter-fixing device (40, 50) according to claim 10.

17. Impression tray with a jaw impression mass, particularly to be used in a device according to one of claims 1 to 13,
characterized in that
the impression tray (30) may be fixed to the upper and/or the lower jaw merely by means of a vacuum.

18. Impression tray according to claim 17, characterized in that a vacuum connection piece (39) is connected to the jaw impression mass (31).

19. Impression tray according to claim 17 or 18, characterized in that the impression tray (30) is connected to a fastening device, especially an upper-jaw cross-plate (27).

20. Impression tray according to one of claims 17 to 19, characterized in that at least one flexible strip (38) may be inserted into the jaw impression mass (31) to form a vacuum channel in the hardened jaw impression mass (31).

21. Impression tray according to one of claims 17 to 20, characterized in that there is associated with one flat side of the impression tray (30) a fixing element for adjoining soft parts, especially for the tongue.

22. Impression tray according to claim 21, characterized in that the fixing element is formed by a vacuum channel.

23. Method of carrying out an operation simulation, characterized in that the calibration point defined as the reference point (37) by the impression tray (30) according to any of claims 17 to 22 is correlated with virtual images generated from a patient-scanning device.

## Revendications

1. Dispositif pour fixer la tête humaine, comprenant:
- une plaque semelle (1),
- au moins un élément d'étayage (4) pouvant être disposé de façon détachable sur la plaque semelle (1), chaque élément d'étayage possédant un support de base (5, 15) pouvant être positionné librement sur la plaque semelle (1),
- une tige de base (7, 17) fixée au support de base (5, 15) et présentant au moins une liaison articulée (9, 19),
- une tige de maintien (8, 18) fixée à la liaison articulée (9, 19) et s'étendant en porte-à-faux, avec prévision d'une plaque transversale de mâchoire supérieure (27) disposée amovible sur l'extrémité de la tige de maintien (8, 18), et
- un porte-empreinte de mâchoire supérieure (30), pouvant être mis en place sur la plaque transversale de mâchoire supérieure (27), qui peut être fixé par dépression à la mâchoire supérieure.

2. Dispositif selon la revendication 1, caractérisé en ce que le porte-empreinte de mâchoire supérieure (30) peut être relié à la plaque transversale de mâchoire supérieure (27) au moyen d'un dispositif de blocage à vis (10, 28) ou une colle.

3. Dipositif selon la revendication 1 ou 2, caractérisé en ce que des éléments à liaisons articulées (9, 19), inclinables suivant plusieurs axes, sous la forme de pieds ou statifs (21) à dispositif de blocage central (20), sont prévus sur les supports de base (5, 15).

4. Dispositif selon une des revendications 1 à 3, caractérisé en ce que la plaque semelle (1) peut être installée de façon réglable sur une table d'opération.

5. Dispositif selon une des revendications 1 à 4, caractérisé en ce que la plaque semelle (1) et/ou des parties de l'élément d'étayage, y compris le porte-empreinte de mâchoire supérieure (30), sont formés au moins partiellement de matériau radio-opaque.

6. Dispositif selon une des revendications 1 à 5, caractérisé en ce que la plaque semelle (1) présente des marquages et/ou des perçages d'enfichage (12) pour repositionner le support de base (5, 15) détaché.

7. Dispositif selon une des revendications 1 à 6, caractérisé en ce que les supports de base (5, 15) sont réalisés comme des supports magnétiques ou comme des supports pneumatiques.

8. Dispositif selon une des revendications 3 à 7, caractérisé en ce que les liaisons articulées (9, 19) des pieds (21) sont blocables par une pression de fluide.

9. Dispositif selon une des revendications 1 à 8, caractérisé en ce que les tiges de base (7, 17) et/ou les tiges de maintien (8, 18) ou les pieds (21) sont réalisés comme des dispositifs de support et de réception d'appareils médicaux, en particulier d'appareillages endoscopiques, écarteurs, surfaces pour poser les mains ou aides stéréotaxiques à la navigation.

10. Dispositif selon une des revendications 1 à 9, caractérisé en ce qu'une fixation antagoniste (40, 50) est prévue, par laquelle, par l'intermédiaire de surfaces (42, 52) réglables, une contre-pression peut être exercée sur le corps humain par rapport au porte-empreinte de mâchoire supérieure (30).

11. Dispositif selon une des revendications 1 à 10, caractérisé en ce qu'un anneau appui-tête (3) ou un appui-tête de conformation anatomique est prévu sur la plaque semelle (1).

12. Dispositif selon une des revendications 1 à 11, caractérisé en ce qu'au moins une tringlerie réglable d'étalonnage (33, 34, 35, 36) pour fixer la position de repères (37) est prévue, tringlerie qui peut être mise en place sur le porte-empreinte de mâchoire supérieure (30) et/ou sur la plaque transversale de mâchoire supérieure (27).

13. Dispositif selon une des revendications 1 à 12, caractérisé en ce que des bandes flexibles (38), auxquelles est coordonnée une tubulure de raccordement (39) de la dépression, peuvent être posées dans le matériau (31) pour empreintes introduit dans le porte-empreinte de mâchoire supérieure (30), bandes qui sont réalisées pour pouvoir être retirées, après le durcissement du matériau (31) pour empreintes, en vue de la formation de canaux de dépression.

14. Procédé pour fixer la tête humaine au moyen d'au moins un élément d'étayage et d'une empreinte de mâchoire fixée à cet élément et adaptée au patient, avec utilisation d'un dispositif selon une des revendications 1 - 13, caractérisé en ce que le porte-empreinte (30), supportant l'empreinte de mâchoire, est appliqué d'abord sous l'effet d'une dépression contre la mâchoire supérieure et/ou la mâchoire inférieure puis fixé.

15. Procédé selon la revendication 14, caractérisé en ce que le patient, muni uniquement du porte-empreinte (30) appliqué par dépression et la tringlerie d'étalonnage (33 - 37) qui y est raccordée, est guidé à travers un dispositif d'exploration fournisseur d'images et que le porte-empreinte (30) est ensuite seulement relié à l'élément d'étayage (4).

16. Procédé pour fixer la tête humaine au moyen d'au moins un élément d'étayage et d'une empreinte de mâchoire fixée à cet élément et adaptée au patient, avec utilisation d'un dispositif selon une des revendications 1 - 13, caractérisé en ce qu'une contre-pression peut être exercée, par rapport au porte-empreinte (30) supportant l'empreinte de mâchoire, au moyen d'une fixation antagoniste (40, 50) selon la revendication 10, présentant des surfaces (42, 52) pour l'application de la contre-pression.

17. Porte-empreinte supportant un matériau pour empreintes de mâchoires, destiné en particulier à être utilisé dans un dispositif selon une des revendications 1 à 13, caractérisé en ce que le porte-empreinte (30) peut être fixé simplement par dépression à la mâchoire supérieure et/ou la mâchoire inférieure.

18. Porte-empreinte selon la revendication 17, caractérisé en ce qu'une tubulure de raccordement (39) de la dépression est reliée au matériau (31) pour empreintes de mâchoires.

19. Porte-empreinte selon la revendication 17 ou 18, caractérisé en ce que le porte-empreinte (30) est relié à un dispositif de fixation, en particulier à une plaque transversale de mâchoire supérieure (27).

20. Porte-empreinte selon une des revendications 17 à 19, caractérisé en ce qu'au moins une bande flexible (38) peut être posée dans le matériau (31) pour empreintes de mâchoires en vue de la formation d'un canal de dépression dans ce matériau (31) après son durcissement.

21. Porte-empreinte selon une des revendications 17 à 20, caractérisé en ce qu'un élément de fixation pour des parties molles limitrophes, en particulier pour la langue, est coordonné à un côté plat du porte-empreinte (30).

22. Porte-empreinte selon la revendication 21, caractérisé en ce que l'élément de fixation est formé par un canal de dépression.

23. Procédé pour réaliser une simulation d'opération, caractérisé en ce que le repère, défini en tant que point fixe (37) par le porte-empreinte (30) selon une des revendications 17 - 22, est corrélé avec des images virtuelles générées à partir d'un dispositif d'exploration de patient.
